# EUROPÄISCHE PATENTSCHRIFT

(11) **EP 2 327 380 B1**
(45) Veröffentlichungstag und Bekanntmachung des Hinweises auf die Patenterteilung: **17.06.2015**
(21) Anmeldenummer: 10189825.2
(22) Anmeldetag: 03.11.2010
(51) Int. Cl.: A61F 2/90

(54) **Stent mit Funktionselementen**
Stent having functional elements
Stent doté d'éléments de fonction

(30) Priorität: 30.11.2009 US 264855 P
(43) Veröffentlichungstag der Anmeldung: 01.06.2011
(73) Patentinhaber: Biotronik VI Patent AG, 6341 Baar (CH)
(72) Erfinder: Gerold, Bodo, 91225, Zellingen (DE); Esperschidt, Dietmar, 90768, Fürth (DE)
(74) Vertreter: Lindner-Vogt, Karin L.

(56) Entgegenhaltungen:
- WO-A1-2004/021929
- WO-A2-2005/122960
- US-A1- 2003 204 245
- US-A1- 2009 005 848
- US-A1- 2009 248 133
- US-B1- 7 572 286

## Beschreibung

Die Erfindung bezieht sich auf ein Verfahren zum Anbringen einer Endoprothese, insbesondere auf eine intraluminale Endoprothese, mit Funktionselementen auf einem Ballonkatheter.

In zunehmendem Maß werden in der modernen Implantationsmedizin Implantate zur Wiedereröffnung und Stützung von Hohlorganen wie zum Beispiel Blutgefäßen, Harnleitern, Gallengängen, Uteri und Bronchien im menschlichen Körper eingesetzt.

Die Implantation von Stents hat sich als eine der wirkungsvollsten therapeutischen Maßnahmen bei der Behandlung von Gefäßerkrankungen etabliert. Stents haben den Zweck, in Hohlorganen eines Patienten eine Stützfunktion zu übernehmen. Stents herkömmlicher Bauart weisen dazu einen Grundkörper auf, der eine Vielzahl von umlaufenden Stützstrukturen z. B. aus metallischen Streben aufweist, der zur Einbringung in den Körper zunächst in einer komprimierten Form vorliegt und am Ort der Applikation aufgeweitet wird. Einer der Hauptanwendungsbereiche solcher Stents ist das dauerhafte oder temporäre Weiten und Offenhalten von Gefäßverengungen, insbesondere von Verengungen (Stenosen) der Herzkranzgefäße. Daneben sind auch Aneurysmenstents bekannt, die eine Stützfunktion für eine beschädigte Gefäßwand bzw. eine Abdichtung von zum Beispiel intrazerebralen Gefäßaussackungen bieten.

Zur Stenose-Behandlung in Blutgefäßen wird in einem medizinischen Eingriff erst eine Ballondilatation durchgeführt und anschließend ein Stent zur Verhinderung einer erneuten Verengung des geweiteten Gefäßes eingesetzt. In einem anderen Verfahren wird die Ballondilatation gleichzeitig mit dem Platzieren des Stents durchgeführt. In einem weiteren Verfahren wird die Verengung des Blutgefäßes allein durch Einsetzen eines selbstexpandierenden Stents beseitigt.

Der Stent besitzt einen Grundkörper aus einem Implantatwerkstoff. Ein Implantatwerkstoff ist ein nicht lebendes Material, das für eine Anwendung in der Medizin eingesetzt wird und mit biologischen Systemen in Wechselwirkung tritt. Grundvoraussetzungen für den Einsatz eines Werkstoffes als Implantatwerkstoff, der bei bestimmungsgemäßem Gebrauch mit der Körperumgebung in Kontakt steht, ist dessen Körperverträglichkeit (Biokompatibilität). Unter Biokompatibilität wird die Fähigkeit eines Werkstoffes verstanden, in einer spezifischen Anwendung eine angemessene Gewebereaktion hervorzurufen. Dies beinhaltet eine Anpassung der chemischen, physikalischen, biologischen und morphologischen Oberflächeneigenschaften eines Implantates an das Empfängergewebe mit dem Ziel einer klinisch erwünschten Wechselwirkung. Die Biokompatibilität des Implantatswerkstoffs ist weiterhin abhängig vom zeitlichen Ablauf der Reaktion des Biosystems, in das implantiert wird. So treten relativ kurzfristig Reizungen und Entzündungen auf, die zu Gewebeveränderungen führen können. Biologische Systeme reagieren demnach in Abhängigkeit von den Eigenschaften des Implantatswerkstoffs in verschiedener Weise. Entsprechend der Reaktion des Biosystems können die Implantatswerkstoffe in bioaktive, bioinerte und biodegradierbare/resorbierbare Werkstoffe unterteilt werden.

Zur Kontrolle während des Vorgangs der Implantation und zur Platzierung des Stents weisen herkömmliche Stents einen Marker aus einem oder mehreren röntgenopaken Materialien auf.

Aus der Druckschrift DE 103 17 241 A1 ist ein Stent mit einem metallischen, radioluzenten Grundgitter und Markerelementen bekannt, die radioopakes Material aufweisen. Mittels Ausschneiden werden in Stegen des Grundgitters des bekannten Stents Ausnehmungen vorgesehen, die als Trägerstruktur wirken und in die später Markerelemente eingeschweißt und von einer Deckschicht aus Siliziumkarbid umschlossen werden. Eine weitere, in dieser Druckschrift offenbarte, allerdings kostenaufwändige Möglichkeit besteht darin, das Grundgitter vollständig aus einem Nitinoldraht mit einer Gold-Seele, die als Röntgenmarker dient, zu fertigen. Dieser mit der Gold-Seele versehene Nitinoldraht bildet den gesamten Endabschnitt des Stents und ist mit dem Grundgitter durch Schweißen verbunden.

Eine ähnliche Lösung wird auch in der Druckschrift DE 100 64 569 A1 beschrieben. Das in dieser Druckschrift offenbarte Verfahren zum Anbringen eines Markerelements an einen Stent sieht vor, dass ein zu verfestigendes, fließ- oder schüttfähiges Material oder Materialgemisch, beispielsweise in Form eines Granulats, in eine Ausnehmung am Grundgitter des Stents eingebracht und dort verfestigt wird. Die Verfestigung dieses Materials erfolgt beispielsweise mittels Sintern.

Zur Unterstützung der Therapie werden seit einiger Zeit Stents mit Medikamenten beschichtet. Die Medikamente dienen entweder der Vermeidung/Verminderung von entzündlichen Reaktionen der Gefäßwände nach dem Eingriff (antiinflammatorisch) oder der überschießenden Proliferation glatter Gefäßmuskelzellen, was zu einer Restenose führen kann. Manche Substanzen dienen auch der beschleunigten Besiedlung des Stents mit Endothelzellen. Dieser gewünschte Effekt beschleunigt das Einwachsen des Stents in die Gefäßwand.

Anordnungen diese Art sind beispielsweise aus der US 6,120,536 bekannt. Dort wird ein Koronarstent offenbart, der eine Polymerbeschichtung, in die Heparin inkorporiert ist und gegebenenfalls eine wirkstofffreie Deckschicht auf der Heparin-inkorporierten Schicht aufweist, umfasst. Des Weiteren sind Koronarstents bekannt, die Rapamycin in einer nicht resorbierbaren polymeren Trägermatrix auf dem Koronarstent aufweisen.

WO 2004/0219259 offenbart einen intraluminalen Stent aus einem ersten Teilstent und einem zweiten Teilstent, wobei der erste Teilstent gegenüber dem zweiten Teilstent verdreht sein kann, um die Eigenschaften des intraluminalen Stents vor oder während der Implantiation in das Körpergefäß zu variieren.

Die in US 2003/204245 offenbarte Erfindung vermeidet, dass ein Patient eine doppelte Dosis eines therapeutischen Wirkstoffes erhält, wenn ein beschichteter Stent mit einem anderen beschichteten Stent im Körpergefäß überlappt. Ein Stent mit einer therapeutischen Beschichtung in einem Bereich wird derart in das Körpergefäß eingesetzt, dass er mit einem zweiten Stent mit einer therapeutischen Beschichtung in dem Bereich überlappt, wo der Stent keine therapeutische Beschichtung aufweist.

WO 2005/122960 offenbart einen rohrförmigen Einsatz für ein Körpergefäß umfassend einen inneren Stent und einen äußeren Stent. Wenigstens ein Teil des inneren Stents ist innerhalb des äußeren Stents angeordnet. Dabei besteht eine substantielle nicht elektrisch leitende Verbindung zwischen inneren und äußeren Stent.

US 7572286 offenbart eine intravaskuläre Stentanordnung zur Implantation in einem Körpergefäß, welches zur Behandlung einer Läsion mit anfälligen Plaque unter Reduktion der der Faserspitzenbelastung ausgestaltet ist. Ein Polymerschlauch verbindet einen ersten und einen zweiten Metallstent.

US 2009/005848 offenbart eine vaskulare Prothese und eine zugehöriges Anordnungsverfahren, welche aus einer Vielzahl modulare, ineinandergreifender Segmente besteht, die über flexible und vorzugsweise arretierbare ineinandergreifende Element verbunden sind. Die Segmente können eine Vielzahl unterschiedliche mechanische Eigenschaften aufweisen und können vom Kliniker über eine Vielzahl mechanischer und chemischer Verbindungen derart angeordnet werden, dass die Prothese für einen spezifischen Patienten oder ein spezifische Anordnung individualisiert ist.

Alle beschriebenen Lösungen haben den Nachteil, dass die zusätzlichen Funktionen, die ein Stent über die Offenhaltung eines Gefäßes hinaus bieten sollte, jeweils fest mit dem Stent verbunden sind. Es besteht nicht die Möglichkeit, den Stent mit seinen zusätzlichen Funktionen - wie Marker oder Medikamentenbeschichtung - zu variieren.

Die Aufgabe der vorliegenden Erfindung ist es daher, eine Endoprothese zur Verfügung zu stellen, die sich aus einem Grundkörper und einem oder mehreren Funktionselementen zusammensetzt. Dabei sollen die Funktionselemente auswählbar sein aus einer Palette von verschiedenen Funktionselementen und abgestimmt werden können auf die verschiedenen Behandlungskonzepte. Gleichzeitig sollte gewährleistet sein, dass sich das bestimmungsgemäße Verhalten des Stents - wie Flexibilität, Recoil und Stützkräfte - nicht nachteilig verändert.

Diese Aufgabe wird erfindungsgemäß gelöst durch das in Anspruch 1 definierte Verfahren zur Anbringung einer Endoprothese, die aus einem röhrenförmigen Grundkörper und mindestens einem Funktionselement besteht auf einem Ballonkatheter. Das mindestens eine Funktionselement ist ebenfalls röhrenförmig und derart an dem Grundkörper angeordnet, dass es den Grundkörper zumindest in Teilbereichen und mindestens teilweise so umschließt, dass es mit dem Grundkörper konzentrisch ausgerichtet ist.

Sowohl der Grundkörper als auch das oder die Funktionselemente bestehen vorzugsweise aus einem metallischen Material aus einem oder mehreren Metallen aus der Gruppe Eisen, Magnesium, Nickel, Wolfram, Titan, Zirkonium, Niob, Tantal, Zink, Silizium, Lithium, Natrium, Kalium, Kalzium, Mangan. In einem weiteren Ausführungsbeispiel bestehen Grundkörper und Funktionselemente aus einem Formgedächtnis-Material aus einem oder mehreren Materialien aus der Gruppe bestehend aus Nickel-Titan-Legierungen und Kupfer-Zink-Aluminium-Legierungen, vorzugsweise aber aus Nitinol. In einem weiteren bevorzugten Ausführungsbeispiel bestehen Grundkörper und Funktionselemente aus Edelstahl, vorzugsweise aus einem Cr-Ni-Fe-Stahl - hier bevorzugt die Legierung 316L - oder einem Co-Cr-Stahl. Ferner kann der Grundkörper des Stents mindestens teilweise aus einem Polymer (z. B. PLLA, PLGA, HA, PU) und/oder einer Keramik bestehen.

In einer bevorzugten Ausführungsform bestehen Grundkörper und/oder Funktionselemente aus einer biodegradierbaren Legierung, ausgewählt aus der Gruppe Magnesium, Eisen, Zink und Wolfram; insbesondere ist der biodegradierbare metallische Werkstoff eine Magnesiumlegierung. Unter Legierung wird vorliegend ein metallisches Gefüge verstanden, deren Hauptkomponente Magnesium, Eisen, Zink oder Wolfram ist. Hauptkomponente ist die Legierungskomponente, deren Gewichtsanteil an der Legierung am höchsten ist. Ein Anteil der Hauptkomponente beträgt vorzugsweise mehr als 50 Gew.%, insbesondere mehr als 70 Gew.%. Ist der Werkstoff eine Magnesiumlegierung, so enthält diese vorzugsweise Yttrium und weitere Seltenerdmetalle, da sich eine derartige Legierung aufgrund ihrer physiko-chemischen Eigenschaften und hohen Biokompatibilität, insbesondere auch seiner Abbauprodukte, auszeichnet. Besonders bevorzugt wird die Magnesiumlegierung WE43. Als biodegradierbar werden Legierungen bezeichnet, bei denen in physiologischer Umgebung ein Abbau stattfindet, der letztendlich dazu führt, dass die gesamte Endoprothese oder der aus dem Werkstoff gebildete Teil der Endoprothese seine mechanische Integrität verliert.

Weitere Ausführungsbeispiele betreffen die unbeschränkten Kombinationsmöglichkeiten von degradierbaren und nicht-degradierbaren Teilen der Endoprothese. In einem Ausführungsbeispiel ist die Endoprothese zusammengesetzt aus einem nicht-degradierbaren Grundkörper und einem oder mehreren degradierbaren Funktionselementen. Vorstellbar ist ebenfalls, dass die Endoprothese aus einem nicht-degradierbaren Grundkörper und mehreren Funktionselementen besteht, wobei einzelne Funktionselemente biodegradierbar sind und andere Funktionselemente nicht-degradierbar. Nicht-degradierbare Funktionselemente können beispielsweise Röntgenmarker sein, die die behandelte Stelle im Gefäß auch dann noch markieren, wenn der Stent längst degradiert ist. Dies kann für die Nachsorge des Patienten von Bedeutung sein.

Durch das erfindungsgemäße modulare Konzept ist es möglich, ein oder mehrere Funktionselemente, auswählbar aus einer größeren Anzahl verschiedener Funktionselemente, mit dem Grundkörper zu verbinden. Dadurch dass die Funktionselemente konzentrisch um den Grundkörper angeordnet sind, werden weder Durchmesser noch Umfang der Endoprothese besonders vergrößert. Gleichzeitig wird durch die Art der Anordnung die erforderlich Flexibilität der Endoprothese nicht beschränkt.

In einer besonders bevorzugten Ausführungsform weist der Grundkörper Aussparungen auf, die mit einem Funktionselement ausfüllbar sind. Hier kann je nach Konzept nur eine Aussparung vorliegen, die mit einem Funktionselement gefüllt wird. Es können jedoch auch mehrere Aussparungen an dem Grundkörper vorgesehen sein, die mit der entsprechenden Anzahl an Funktionselementen ausgefüllt werden. Durch die Anordnung der Funktionselemente in den Aussparungen ist eine besonders hohe Flexibilität der Endoprothese gewährleistet. Gleichzeitig ist bei dieser Lösung der Umfang bzw. der Durchmesser der Endoprothese nicht vergrößert. Besonders bevorzugt sind eine oder zwei Aussparungen, die sich jeweils nahe der Enden der Endoprothese in axialer Richtung befinden.

Der Grundkörper und das Funktionselement bestehen aus einer Gitterstruktur. Die Gitterstruktur kann ein offen-zelliges oder ein geschlossen-zelliges Design sein. Die intraluminale Endoprothese kann z. B. ein Stent-System sein, bei dem der Grundkörper ein (Basis-)Stent ist, an dem das Funktionselement angeordnet ist. Hierbei besteht der (Basis-)Stent, also der Grundkörper, aus einer gitterartigen Umfangswandung, die es erlaubt, den (Basis-)Stent in einem komprimierten (gecrimpten) Zustand mit kleinem Außendurchmesser bis zur zu behandelnden Engstelle des jeweiligen Gefäßes einzuführen und dort beispielsweise mit Hilfe eines Dilatationsballonkatheters soweit aufzuweiten, dass das Gefäß den gewünschten, vergrößerten Innendurchmesser aufweist. Es kann sich jedoch auch um einen selbstexpandierenden Stent handeln. Dann sind sowohl der Grundkörper als auch die Funktionselemente beispielsweise aus einer Formgedächtnislegierung, wie z.B. Nitinol, hergestellt oder sie weisen ein selbstexpandierendes Stent-Design (z. B. Wallstent und/oder Coil-Design) auf.

Ebenso besteht das Funktionselement aus einer Gitterstruktur, die im Falle einer Anbringung auf einem Grundkörper, der als (Basis-)Stent fungiert, dieselben Eigenschaften wie der Stent aufweist. Das bedeutet, dass das Funktionselement ebenfalls auf einen kleinen Außendurchmesser komprimiert (gecrimpt) und am Behandlungsort dilatiert werden kann.

Die Anbringung von Grundkörper und Funktionselement auf einem Ballonkatheter erfolgt in zwei aufeinander folgenden Schritten. Im ersten Schritt wird der Grundkörper über den Ballon geschoben und dort gecrimpt. In einem zweiten Schritt wird das Funktionselement über diese Anordnung von Ballon und Grundkörper gebracht und anschließend gecrimpt. Dieses System bringt den Vorteil, dass durch das außenliegende, ebenfalls mit einem bestimmten Druck gecrimpte Funktionselement der darunterliegende Grundkörper stärker auf den Ballon gepresst wird. Die Haltekraft der Endoprothese bzw. des (Basis-)Stents auf dem Ballon ist damit erhöht; die Gefahr des Verrutschens des gesamtem Stent-Systems auf dem Ballon oder gar die Gefahr des kompletten Stentsystemverlusts ist damit stark verringert.

In einer bevorzugten Ausführungsform haben Grundkörper und Funktionselement dieselbe Gitterstruktur. Der Grundkörper kann beispielsweise eine der bekannten Helix- bzw. Mäandermuster eines Stents aufweisen; und das Funktionselement nimmt diese Gitterstruktur auf. In dieser bevorzugten Ausführungsform kann der Grundkörper ebenfalls die beschriebenen Aussparungen aufweisen, die durch ein oder mehrere Funktionselemente gefüllt werden können. Diese Aussparungen befinden sich bevorzugt an den Enden der Endoprothese in axialer Richtung und können beispielsweise das vorletzte Ringsegment der Gitterstruktur ersetzen. Man erhält dann eine formschlüssige Endoprothese bzw. ein Stent-System, das nur wenige Überlappungen der Struktur aufweist und dadurch sowohl hervorragend komprimierbar als auch besonders flexibel ist.

Überlappungen von Grundkörper und Funktionselementen können ausgeglichen werden durch entsprechend dünnere Strukturen an den Stellen der Überlappungen. So kann beispielsweise die Gitterstruktur sowohl des Grundkörpers als auch der Funktionselemente an den Orten der Überlappung dünnere Stegbreiten aufweisen.

Die Implantation einer Endoprothese bzw. der Vorgang der Positionierung und Expansion des Stent-Systems während der Prozedur und die abschließende Lage des Stents im Gewebe nach Beendigung der Prozedur muss durch den Kardiologen überwacht werden. Dies kann durch bildgebende Verfahren, wie z. B. durch Röntgenuntersuchungen erfolgen. Daher dient in einer bevorzugten Ausführungsform das Funktionselement als Marker. Zu diesem Zweck enthält das Funktionselement radioopakes Material bzw. besteht das Funktionselement daraus. Durch seine, den gesamten Umfang der röhrenartigen Gestalt des Grundkörpers umschreibende Form überdeckt der Marker eine relativ große Fläche und ist unter den bekannten Bildgebungsverfahren besonders gut zu erkennen.

Für degradierbare Endoprothesen bieten sich gefüllte Polymere (z. B. Poly-L-Lactid, Poly-D,L-Lactid, Triblockcopolymere, Polyorthoester, Polysaccharide) oder degradierbare Metalle bzw. Metalllegierungen (z. B. Fe, Zn, Mg, W, FeMn-Legierungen, MgAl-Legierungen wie AZ31, AZ80, AZ91; Magnesium-Seltene-Erden-Legierungen wie WE43) an. Für nicht-degradierbare Endoprothesen kann das radioopake Material zusammengesetzt sein aus ein oder mehreren Elementen der Gruppe bestehend aus Ta, W, Au, Ir, Pt und deren Legierungen.

In einem weiteren bevorzugten Ausführungsbeispiel enthält das Funktionselement einen oder mehrere Wirkstoffe. Das Funktionselement kann mit einem oder mehreren Wirkstoffen beschichtet sein oder das Material des Funktionselementes ist mit dem Wirkstoff durchsetzt. Ein Wirkstoff im Sinne der Erfindung ist eine pflanzliche, tierische oder synthetisierte pharmazeutisch aktive Substanz, die in geeigneter Dosierung als Therapeutikum zur Beeinflussung von Zuständen oder Funktionen des Körpers, als Ersatz für natürlich vom menschlichen oder tierischen Körper erzeugte Wirkstoffe sowie zur Beseitigung oder zum Unschädlichmachen von Krankheitserregern oder Körperfremdstoffen Einsatz findet. Die Freisetzung der Substanz in der Implantatsumgebung hat einen positiven Effekt auf den Heilungsverlauf bzw. wirkt pathologischen Veränderungen des Gewebes infolge des chirurgischen Eingriffes entgegen.

Derartige pharmazeutisch aktive Substanzen weisen beispielsweise eine antiinflammatorische und/oder antiproliferative und/oder spasmolytische Wirkung auf, wodurch beispielsweise Restenosen, Entzündungen oder (Gefäß-)Spasmen vermieden werden können. Derartige Substanzen können in besonders bevorzugten Ausführungsbeispielen aus einer oder mehreren Substanzen der Wirkstoffgruppe der Kalziumkanalblocker, der Lipidregulatoren (wie beispielsweise Fibrate), der Immunsuppressiva, der Calcineurininhibitoren (wie beispielsweise Tacrolimus), der Antiflogistika (wie beispielsweise Cortison oder Dichlofenac), der Antiinflammatorika (wie beispielsweise Imidazole), der Antiallergika, der Oligonucleotide (wie beispielsweise dODN), der Östrogene (wie beispielsweise Genistein), der Endothelbildner (wie beispielsweise Fibrin), der Steroide, der Proteine, der Hormone, der Insuline, der Zytostatika, der Peptide, der Vasodilatatoren (wie beispielsweise Sartane), der antiproliferativ wirkenden Stoffe der Taxole oder Taxane, hier vorzugsweise Paclitaxel oder Sirolimus und seine Derivate, sowie aus solchen lipophilen Substanzen, die eine Gewebekalzifizierung oder die Neointimabildung hemmen, wie Vitamin A- und D-Derivate und Phyllochinon/Menachinon- (Vitamin K)-Derivate, bestehen.

Nach einer weiteren bevorzugten Ausführungsform weist die Endoprothese eine oder mehrere Beschichtungen auf. Es können jeweils der Grundkörper und die Funktionselemente einzeln, getrennt voneinander und mit verschiedenen Materialen beschichtet sein. Oder aber Grundkörper und Funktionselemente weisen dieselbe durchgehende Beschichtung auf. Die Beschichtungen können jeweils biodegradierbar oder auch permanent, also nichtdegradierbar sein.

Es kann sich um Beschichtungen handeln, die Wirkstoffe aufnehmen oder transportieren können. Die Beschichtungen können jedoch auch die Aufgabe haben, die Endoprothese oder Teile hiervon vor Abrieb zu schützen, der auf die Endoprothese wirken kann, sei es durch die Handhabung der Endoprothese außerhalb des Körpers, durch den Vorgang der Implantation oder durch die physiologische Umgebung im menschlichen oder auch tierischen Körper. Diese Beschichtung kann beispielsweise bestehen aus Parylene, Teflon, DLC, SiC, Polyurethanen, Polyesterimiden, Polyimiden oder es kann sich um eine durch Nitrierung erhaltene Beschichtung handeln.

Darüber hinaus kann die Beschichtung auch erforderlich oder gewünscht sein, um galvanische Effekte zwischen zwei metallischen Bestandteilen der Endoprothese zu unterdrücken. Dies kann der Fall sein bei z. B. einem Grundkörper enthaltend Magnesium und einem Funktionselement enthaltend Gold. Hier bieten sich Beschichtungen aus z. B. Parylene, Teflon, DLC, SiC, Silikon, Polyurethanen, Polyesterimiden und/oder Polyimiden an.

Umgekehrt kann jedoch auch gerade dieser galvanische Effekt in Form einer Kontaktkorrosion gewünscht sein, so dass eine Beschichtung gewählt wird, die z. B. den Grundkörper kathodisch schützt, indem das oder die Funktionselemente elektrisch leitend mit dem Grundkörper verbunden werden. Beispielsweise kann der Grundkörper aus Titan-Zink (einer Zink-Legierung mit wenig (ca. 0,5-3%) Titan) oder aber aus einer Eisen-Mangan-Legierung bestehen. Das oder die Funktionselemente können beispielsweise aus einer Magnesium-Legierung, wie z. B. WE43, bestehen.

Um Wirkstoffe aufzunehmen bzw. zu transportieren, sind Beschichtungen aus einem oder mehreren unterschiedlichen Polymeren zweckmäßig. Bevorzugt sind Polymere aus der Gruppe bestehend aus:
- nicht-degradierbare (permanente) Polymere:
   Polypropylen; Polyethylen; Polyvinylchlorid; Polyacrylate, vorzugsweise Polyetyl- und Polymethylacrylate, Polymethylmetacrylat; Polymethyl-co-ethyl-acrylat, Ethylen/Ethylacrylat etc.; Polytetrafluorethylen, vorzugsweise Ethylen/Chlortrifluorethylen Copolymere, Ethylen/Tretrafluorethylen Copolymere; Polyamide, vorzugsweise Polyamidimid, PA-11, -12, -46, -66 etc.; Polyetherimid; Polyethersulfon; Poly(iso)butylen;
   Polyvinylchlorid; Polyvinylfluorid; Polyvinylalkohol; Polyurethan; Polybuthylenterephthalat; Silikone; Polyphosphazen; Polymerschäume, vorzugsweise aus Carbonaten, Styrolen etc.; sowie Copolymere und Blends der aufgezählten Klassen, bzw. der Klasse der Thermoplaste im Allgemeinen
- biodegradierbare Polymere:
   Poly-dioxanon; Polyglycolid; Polycaprolacton; Polylactide, vorzugsweise Poly-L-Lactid, Poly-D,L-Lactid, und Copolymere sowie Blends wie Poly(L-Lactid-co-glycolid), Poly(D,L-lactid-co-glycolid), Poly(L-Lactid-co-D,L-Lactid), Poly(1-Lactid-co-trimethylen carbonat); Triblockcopolymere; Polyorthoester; Polysaccharide, vorzugsweise Chitosan, Levan, Hyaluronsäure, Heparin, Dextran, Cellulose, Chondroitinsulfat etc.; Polyhydroxyvalerat; Ethylvinylacetat; Polyethylenoxid; Polyphosphorylcholin; Peptide, vorzugsweise Fibrin, Albumin, Polyhydroxybuttersäure, vorzugsweise ataktisch, isotaktisch, syndiotaktisch sowie deren Blends.

Besonders bevorzugt sind Polylactide, vorzugsweise Poly-L-Lactid, Poly-D,L-Lactid, und Copolymere sowie Blends wie Poly(L-Lactid-co-glycolid), Poly(D,L-lactid-co-glycolid), Poly(L-Lactid-co-D,L-Lactid), Poly(1-Lactid-co-trimethylen carbonat), und Polysaccharide, vorzugsweise Chitosan, Levan, Hyaluronsäure, Heparin, Dextran, Cellulose, Chondroitinsulfat, sowie Polypeptide, vorzugsweise Fibrin und Albumin.

Für spezielle medizinische Anwendungen ist es möglich, die Beschichtungen der Funktionselemente mit deren inkorporierten Wirkstoffen so zu realisieren, dass die Endoprothese eine auf die Behandlung des Patienten abgestimmte Wirkstoffkinetik aufweist. So kann zum Beispiel ein Teil der Funktionselemente Beschichtungen mit Wirkstoffen aufweisen, bei denen eine schnelle Kinetik gewünscht ist, d. h. bei denen die Wirkstoffe während oder kurze Zeit nach der Implantation freigesetzt werden sollen. Ein anderer Teil der Funktionselemente kann Beschichtung und Wirkstoff mit einer langsamen Kinetik kombinieren, so dass der Wirkstoff entweder langsamer oder aber zu einem späteren Zeitpunkt nach der Implantation freigesetzt wird. So kann beispielsweise eine eher schnelle Kinetik mit der Einbettung des Wirkstoffes (z. B. Paclitaxel) in PLGA erzeugt werden; und eine langsamere Kinetik mit der Einbettung des Wirkstoffes in PLLA.

Die Herstellung sowohl des Grundkörpers als auch der Funktionselemente kann auf alle herkömmlichen Arten erfolgen: Laserstrahlschneiden, Extrusion, Schweißen, Gießen, Biegen, Börteln, Falten, Nieten, Hart- oder Weichlöten.

Auch das Crimpen erfolgt auf herkömmlichem Weg. Zur exakten Positionierung von Grundkörper und Funktionselementen können speziell angefertigte Positioniereinrichtungen verwendet werden.

Der beachtliche Vorteil des modularen Konzeptes ist die Möglichkeit der individuellen Anpassung der Art der Funktionselemente an die Anforderungen der Behandlung. So kann die Anzahl und die Art der Funktionselemente, die an dem ebenfalls individuell gewählten Grundkörper angebracht sind, an den jeweiligen Patienten und seine Beschwerden, Krankheiten, medikamentösen Erfordernissen usw. angepasst werden. Es kann ohne Schwierigkeiten auf Allergien und Unverträglichkeiten Rücksicht genommen werden. Auch kann - wenn nötig erst kurz vor dem Eingriff - auf Größe, Statur und Verfassung des Patienten Rücksicht genommen werden, wenn z. B. das medizinische Personal in die Lage versetzt wird, die Zusammensetzung von Grundkörper und jeweils notwendigen Funktionselementen kurz vor dem operativen Eingriff zu entscheiden und die Endoprothese nach dem Baukastenprinzip zusammenzusetzen.

Gleichzeitig ist dieses modulare Konzept platzsparend, da nicht jede denkbare Endoprothese im Lager vorgehalten werden muss, sondern nur die Einzelteile, die bei Bedarf entnommen werden. Aus den gleichen Gründen ist die erfindungsgemäße Lösung kostensenkend für Hersteller und Verbraucher.

Die Erfindung wird anhand eines Ausführungsbeispiels in Verbindung mit den Fig. 1 bis 5 näher erläutert.
- Fig. 1: zeigt schematisch den Grundkörper (10) der Endoprothese (1) in Form eines (Basis-)Stents mit einer Aussparung (12).
- Fig. 2: zeigt schematisch ein Funktionselement (20).
- Fig. 3: zeigt schematisch die gesamte Endoprothese (1) in Form eines Stent-Systems.
- Fig. 4: zeigt Grundkörper (10) und Funktionselement (20) in der Ansicht entlang der Längsachse (A) der Endoprothese (1).
- Fig. 5: zeigt ebenfalls Grundkörper (10) und Funktionselement (20) in der Ansicht entlang der Längsachse (A) der Endoprothese (1).

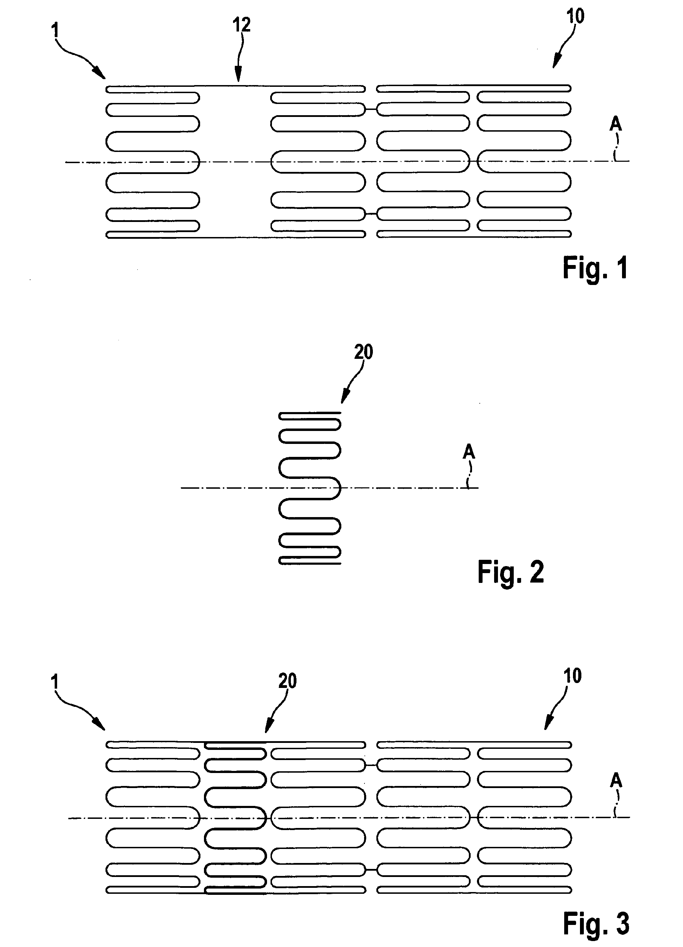

Der in der Fig. 1 gezeigte Grundkörper (10) und das in der Fig. 2 gezeigte Funktionselement (20) sind erfindungsgemäß röhrenförmig. Der Grundkörper weist eine Aussparung (12) auf. Die in Fig. 3 gezeigte Endoprothese (1) setzt sich zusammen aus dem röhrenartigen Grundkörper (10) und dem ebenfalls röhrenartigen Funktionselement(20). Das Funktionselement (20) ist dabei in der Aussparung (12) des Grundkörpers (10) angeordnet. Das Funktionselement (20) umschließt den Grundkörper (10) zumindest in Teilbereichen und mindestens teilweise so, dass es mit dem Grundkörper (10) konzentrisch ausgerichtet ist.

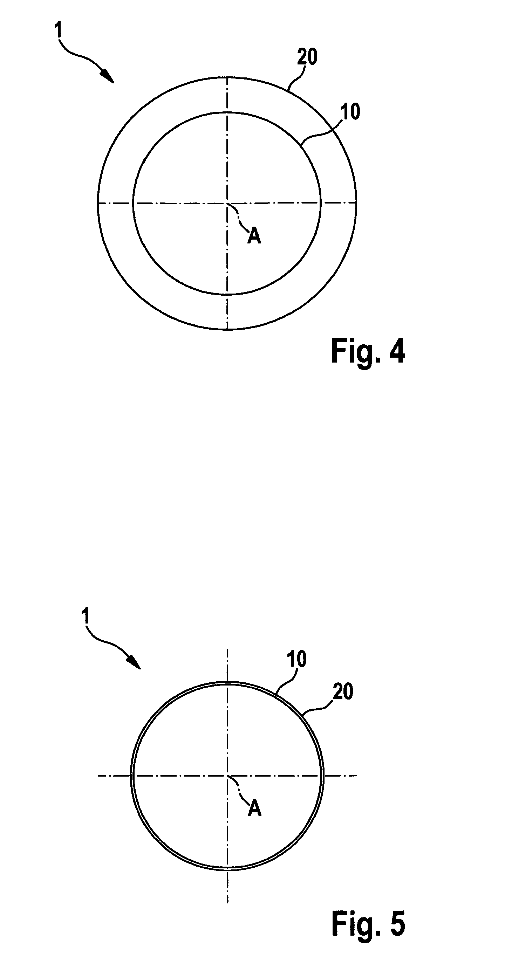

In den Fig. 4 und 5 ist die konzentrische Ausrichtung dargestellt. Um die Längsachse (A), die senkrecht aus der Ebene weist, liegen innen der Grundkörper (10) und außen ein Funktionselement (20). In Fig. 4 ist der Grundkörper (10) bereits gecrimpt worden, d.h. er liegt in einer komprimierten Form mit kleineren Außendurchmesser vor. Im Außenbereich des Grundkörpers (10) ist das Funktionselement (20) gezeigt. Es ist konzentrisch um die Grundkörper (10) mit der gemeinsamen Achse (A) angeordnet. In Fig. 4 ist das Funktionselement (20) noch ungecrimpt und hat daher einen größeren Außendurchmesser als der Grundkörper (10).

In Fig. 5 sind Grundkörper (10) und Funktionselement (20) gleichermaßen gecrimpt. Besonders bevorzugt ist die Ausführungsform mit Aussparungen (12). Füllt das Funktionselement (20), wie hier angenommen, die Aussparung (12) im Grundkörper (10) aus, haben nach der Crimpung Grundkörper (10) und Funktionselement (20) denselben geringen Durchmesser.

### Bezugszeichenliste:

- 1: Endoprothese
- 10: Grundkörper
- 12: Aussparung
- 20: Funktionselement

- A: radiale Achse

## Patentansprüche

1. Verfahren zum Anbringen einer Endoprothese, insbesondere intraluminale Endoprothese, auf einem Ballonkatheter, wobei die Endoprothese aus
- einem röhrenförmigen Grundkörper und
- mindestens einem Funktionselement, besteht und,
wobei das mindestens eine Funktionselement ebenfalls röhrenförmig ist und derart an dem Grundkörper angeordnet ist, dass das mindestens eine Funktionselement den Grundkörper zumindest in Teilbereichen und mindestens teilweise so umschließt, dass es mit dem Grundkörper konzentrisch ausgerichtet ist,
**dadurch gekennzeichnet, dass**
in einem ersten Schritt der Grundkörper über den Ballon geschoben und dort gecrimpt wird und
in einem zweiten Schritt das Funktionselement über dieses Anordnung von Ballon und Grundkörper gebracht und anschließend gecrimpt wird, und
Grundkörper und Funktionselement aus einer Gitterstruktur bestehen.

2. Verfahren nach Anspruch 1, **dadurch gekennzeichnet, dass** der Grundkörper eine oder mehrere Aussparungen aufweist, wobei die eine oder mehreren Aussparungen derart ausgestaltet sind, dass sie durch das Funktionselement ausfüllbar sind.

3. Verfahren nach einem der Ansprüche 1 oder 2, **dadurch gekennzeichnet, dass** der Grundkörper und/oder das mindestens eine Funktionselement eine Gitterstruktur aufweisen.

4. Verfahren nach Anspruch 3, **dadurch gekennzeichnet, dass** der Grundkörper und das mindestens eine Funktionselement dieselbe Gitterstruktur aufweisen, wobei bevorzugt der Grundkörper ein Helix- bzw. Mäandermuster aufweist und das Funktionselement diese Gitterstruktur aufnimmt.

5. Verfahren nach einem der Ansprüche 1 bis 4, **dadurch gekennzeichnet, dass** das mindestens eine Funktionselement röntgenopakes Material enthält.

6. Verfahren nach einem der Ansprüche 1 bis 5, **dadurch gekennzeichnet, dass** das mindestens eine Funktionselement einen oder mehrere Wirkstoffe enthält.

7. Verfahren nach einem der Ansprüche 1 bis 6, **dadurch gekennzeichnet, dass** der Grundkörper und/oder das mindestens eine Funktionselement eine oder mehrere Beschichtungen aufweisen.

8. Verfahren nach einem der Ansprüche 1 bis 7, **dadurch gekennzeichnet, dass** der Grundkörper ein Stent ist.

9. Verfahren nach einem der Ansprüche 1 bis 8, **dadurch gekennzeichnet, dass** die Endoprothese ein Stent ist.

## Claims

1. A method for attaching an endoprosthesis, in particular an intraluminal endoprosthesis, to a balloon catheter, wherein the endoprosthesis consists of
- a tubular main body and
- at least one function element, and
wherein the at least one function element is also tubular and is arranged on the main body in such a way that the at least one function element surrounds the main body at least in partial areas and does so at least partially, so that it is oriented concentrically with the main body,
**characterised in that**
in a first step the main body is advanced over the balloon and crimped there, and
in a second step the function element is brought over this arrangement of balloon and main body and then crimped, and
the main body and function element consist of a mesh structure.

2. The method according to Claim 1, **characterised in that** the main body has one or more recesses, wherein the one or more recesses are designed so that they can be filled with the function element.

3. The method according to one of Claims 1 or 2, **characterised in that** the main body and/or the at least one function element have/has a mesh structure.

4. The method according to Claim 3, **characterised in that** the main body and the at least one function element have the same mesh structure, wherein the main body preferably has a helical and/or meandering pattern, and the function element receives this mesh structure.

5. The method according to one of Claims 1 to 4, **characterised in that** the at least one function element contains radiopaque material.

6. The method according to one of Claims 1 to 5, **characterised in that** the at least one function element contains one or more active ingredients.

7. The method according to one of Claims 1 to 6, **characterised in that** the main body and/or the at least one function element have/has one or more coatings.

8. The method according to one of Claims 1 to 7, **characterised in that** the main body is a stent.

9. The method according to one of Claims 1 to 8, **characterised in that** the endoprosthesis is a stent.

## Revendications

1. Procédé de mise en place d'une endoprothèse, notamment, une endoprothèse intraluminale, sur un cathéter à ballonnet, dans lequel l'endoprothèse est constituée
- d'un corps de base en forme de tube, et
- d'au moins un élément fonctionnel,
où l'au moins un élément fonctionnel est également de forme tubulaire et est disposé sur le corps de base de telle façon que l'au moins un élément fonctionnel entoure le corps de base, au moins sur des zones partielles, et au moins partiellement, de sorte qu'il est orienté concentriquement par rapport au corps de base,
**caractérisé en ce que**
dans une première étape, le corps de base est glissé par-dessus le ballonnet et est serti à cet endroit, et
dans une deuxième étape, l'élément fonctionnel est passé pardessus cet agencement du ballonnet et du corps de base et est ensuite serti, et
le corps de base et l'élément fonctionnel sont constitués d'une structure en mailles.

2. Procédé selon la revendication 1, **caractérisé en ce que** le corps de base présente un ou plusieurs évidements, où l'un ou plusieurs évidements sont conçus de telle façon qu'ils peuvent être comblés par l'élément fonctionnel.

3. Procédé selon l'une des revendications 1 ou 2, **caractérisé en ce que** le corps de base et/ou l'au moins un élément fonctionnel présentent une structure en mailles.

4. Procédé selon la revendication 3, **caractérisé en ce que** le corps de base et l'au moins un élément fonctionnel présentent la même structure en mailles, où le corps de base présente de préférence un motif en hélice, respectivement un motif en méandres, et l'élément fonctionnel adopte cette structure en mailles.

5. Procédé selon l'une des revendications 1 à 4, **caractérisé en ce que** l'au moins un élément fonctionnel contient un matériau opaque aux rayons X.

6. Procédé selon l'une des revendications 1 à 5, **caractérisé en ce que** l'au moins un élément fonctionnel contient une ou plusieurs matières actives.

7. Procédé selon l'une des revendications 1 à 6, **caractérisé en ce que** le corps de base et/ou l'au moins un élément fonctionnel présentent un ou plusieurs revêtements.

8. Procédé selon l'une des revendications 1 à 7, **caractérisé en ce que** le corps de base est une prothèse endovasculaire.

9. Procédé selon l'une des revendications 1 à 8, **caractérisé en ce que** l'endoprothèse est une prothèse endovasculaire.
